Europäisches Patentamt

European Patent Office

Office européen des brevets

(19)

(11) **EP 0 924 303 A2**

(12) **EUROPÄISCHE PATENTANMELDUNG**

(43) Veröffentlichungstag:
23.06.1999 Patentblatt 1999/25

(51) Int. Cl.$^6$: **C12Q 1/68**

(21) Anmeldenummer: 98120922.4

(22) Anmeldetag: 04.11.1998

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 07.11.1997 DE 19749163

(71) Anmelder: **Roche Diagnostics GmbH**
**68298 Mannheim (DE)**

(72) Erfinder:
• **Huber, Erasmus Dr.**
**86923 Finning (DE)**
• **Bergmann, Frank Dr.**
**82393 Iffeldorf (DE)**
• **Seidel, Christoph Dr.**
**82362 Weilheim (DE)**
• **Heindl, Dieter Dr.**
**82327 Tutzing (DE)**

(54) **Verfahren zum Nachweis von Nukleinsäuren**

(57) Ein Verfahren zum Nachweis einer Nukleinsäure mit Hilfe eines Konjugats aus einem nukleinsäurebindenden Rest und einer chemischen Gruppe,
welche selbst nicht an die nachzuweisende Nukleinsäure bindet, aber bei Bindung des Konjugats an die
Nukleinsäure eine neue, nachweisbare Eigenschaft
erhält, ermöglicht eine homogene Versuchsführung.

EP 0 924 303 A2

# SEQUENZPROTOKOLL

(1) ALLGEMEINE ANGABEN:

    (i) ANMELDER:
        (A) NAME: Boehringer Mannheim GmbH
        (B) STRASSE: Sandhferstr. 116
        (C) ORT: Mannheim
        (E) LAND: DE
        (F) POSTLEITZAHL: 68305
        (G) TELEFON: 0621 759 4348
        (H) TELEFAX: 0621 759 4457

    (ii) BEZEICHNUNG DER ERFINDUNG: Verfahren zum Nachweis von Nukleinsaeuren

    (iii) ANZAHL DER SEQUENZEN: 2

    (iv) COMPUTER-LESBARE FASSUNG:
        (A) DATENTRÄGER: Floppy disk
        (B) COMPUTER: IBM PC compatible
        (C) BETRIEBSSYSTEM: PC-DOS/MS-DOS
        (D) SOFTWARE: PatentIn Release #1.0, Version #1.30 (EPA)

(2) ANGABEN ZU SEQ ID NO: 1:

    (i) SEQUENZKENNZEICHEN:
        (A) LÄNGE: 24 Basenpaare
        (B) ART: Nucleotid
        (C) STRANGFORM: Einzelstrang
        (D) TOPOLOGIE: linear

    (ii) ART DES MOLEKÜLS: Sonstige Nucleinsäure
        (A) BESCHREIBUNG: /desc = "Oligodeoxyribonukleotid"

    (xi) SEQUENZBESCHREIBUNG: SEQ ID NO: 1:

CTGTAACAAC AAGTCAGGTT GCGC                                24

(2) ANGABEN ZU SEQ ID NO: 2:

    (i) SEQUENZKENNZEICHEN:
        (A) LÄNGE: 24 Basenpaare
        (B) ART: Nucleotid
        (C) STRANGFORM: Einzelstrang
        (D) TOPOLOGIE: linear

    (ii) ART DES MOLEKÜLS: Sonstige Nucleinsäure
        (A) BESCHREIBUNG: /desc = "Oligodeoxyribonukleotid"

    (xi) SEQUENZBESCHREIBUNG: SEQ ID NO: 2:

GTACTAGAGG ACTTACCTCT TCCC                                24

**Beschreibung**

Gegenstand der Erfindung sind ein Verfahren zum Nachweis einer Nukleinsäure in einer Probe mit Hilfe einer speziellen Sonde, ein Reagenz, welches diese Sonde enthält, sowie ein Reagenzkit, welches die Sonde und weitere Nachweisreagenzien enthält.

Nach der Entwicklung der Polymerase-Kettenreaktion (PCR, EP-B-0 201 184, US-A-4,683,202) kam es in den letzten zehn Jahren zu bedeutenden Fortschritten in der Genomforschung. Die Aufklärung vieler viraler Nukleinsäure-Sequenzen in Verbindung mit Fortschritten in der PCR-Technik führte zur Entwicklung von Diagnoseverfahren für Infektionskrankheiten auf DNA-Ebene. Die mit Hilfe der PCR beliebig vervielfältigbaren Nukleinsäurestränge werden dabei mit Hilfe heterogener Nachweismethoden detektiert. Hierzu muß das Amplifikat spezifisch über eine markierte Fangsonde an die Oberfläche einer festen Phase gebunden und anschließend über eine zweite Markierung detektiert werden. Der Nachweis erfolgt dabei entweder über die ebenfalls spezifische Reaktion mit einer markierten Detektorsonde oder durch Einbau weiterer markierter Moleküle während der Amplifikationsreaktion. Entsprechende Markierungsverfahren sind entwickelt (Non-radioactive Labelling and Detection of Biomolecular; Kessler (Ed.), Springer-Verlag 1992). Alternativ kann der Nachweis auch über markierte Antikörper, die gegen das Amplifikat gerichtet sind, erfolgen.

Alle der oben beschriebenen Nachweismethoden sind Mehrschrittverfahren, in deren Verlauf mindestens ein Trennschritt an der Grenzschicht zwischen fester und flüssiger Phase erfolgen muß. Solche heterogene Reaktionssysteme sind in der Regel geschwindigkeitslimitiert und folglich zeitaufwendig. Es besteht deshalb ein Bedarf, solche Nachweise in homogener, flüssiger Phase zu führen.

Gegenstand der Erfindung ist ein Verfahren zum Nachweis einer Nukleinsäure in einer Probe enthaltend die Schritte

- Behandlung der Probe mit einem Konjugat aus einer chemischen Gruppe, welche selbst nicht an die nachzuweisende Nukleinsäure bindet, aber bei Bindung des Konjugats an die Nukleinsäure eine neue, nachweisbare Eigenschaft erhält, und einem nukleinsäurebindenden Rest unter Bedingungen, bei denen das Konjugat an die nachzuweisende Nukleinsäure bindet, und

- Bestimmung der neuen Eigenschaft der chemischen Gruppe als Zeichen der Anwesenheit oder der Menge der nachzuweisenden Nukleinsäure in der Probe.

Ein weiterer Gegenstand ist ein Reagenz enthaltend das Konjugat und ein entsprechender Reagenzkit.

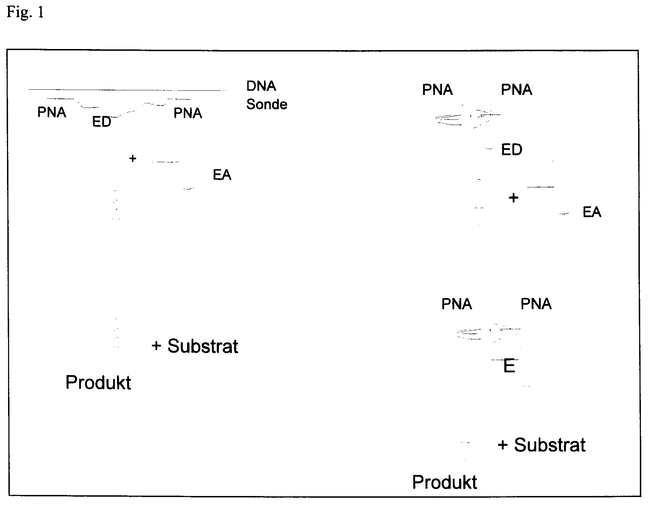

In Fig. 1 ist eine bevorzugte Ausführungsform des Verfahrens schematisch gezeigt.

Bei dem erfindungsgemäßen Verfahren handelt es sich um eine spezielle Ausführungsform der sogenannten Hybridisierungstests, die in ihren Grundzügen dem Fachmann auf dem Gebiet der Nukleinsäurediagnostik bekannt sind. Soweit experimentelle Details im folgenden nicht ausgeführt sind, wird dazu vollinhaltlich auf "Nucleic acid hybridisation", Herausgeber B.D. Hames und S.J. Higgins, IRL Press, 1986, z. B. in den Kapiteln 1 (Hybridisation Strategy), 3 (Quantitative Analysis of Solution Hybridisation) und 4 (Quantitative Filter Hybridisation), Current Protocols in Molecular Biology, Ed. F.M. Ausubel et al., J. Wiley and Son, 1987, und Molecular Cloning, Ed. J. Sambrook et al., CSH, 1989, Bezug genommen. Zu den bekannten Methoden gehört auch die chemische Synthese von modifizierten und unmodifizierten Oligonukleotiden, die Spaltung von Nukleinsäuren mittels Restriktionsenzymen, die Auswahl von Hybridisierungsbedingungen, durch welche eine Spezifität erreicht werden kann, die vom Ausmaß der Homologie zwischen den zu hybridisierenden Nukleinsäuren, deren GC-Gehalt und deren Länge abhängt.

Unter Analytnukleinsäuren sind Nukleinsäuren jeglichen Ursprungs zu verstehen, beispielsweise Nukleinsäuren viroiden, viralen, bakteriellen oder zellulären Ursprungs. Sie können in Lösung, Suspension, aber auch an Festkörpern fixiert oder in zellhaltigen Medien, Zellabstrichen, fixierten Zellen, Gewebsschnitten oder fixierten Organismen vorliegen. Bevorzugt liegen die Nukleinsäuren in Lösung vor.

Die Reaktionssequenz wird meist gestartet durch Verfügbarmachung der Analytnukleinsäure mit entsprechenden Reagentien. Hierbei können sowohl Veränderungen des pHs (alkalisch), Hitze, Wiederholung extremer Temperaturveränderungen (Einfrieren/Auftauen), Veränderung der physiologischen Wachstumsbedingungen (Osmotischer Druck), Einwirkung von Detergentien, chaotropen Salzen oder Enzymen (z.B. Proteasen, Lipasen), alleine oder in Kombination angewendet werden.

Beispielsweise kann ein Verfahren zum Nachweis eines speziellen Virus in einer Körperflüssigkeit (z.B. Serum) als ersten Schritt die Lyse der Virushülle enthalten. Verfahren zur Lyse von Zellwänden sind dem Fachmann bekannt. Beispielsweise kann die Lyse durch Behandlung mit Alkalihydroxydlösungen vorgenommen werden. Auch der Zusatz von Hilfsstoffen, z.B. Detergentien, ist möglich. Bei einem Nachweis von Bakterien, beispielsweise in Lebensmitteln, könnten dem erfindungsgemäßen Verfahren ebenfalls mehrere Schritte vorgeschaltet werden. Im allgemeinen werden Bakterienproben ggf. nach in vivo Vermehrung der Bakterien unter Bedingungen, welche die Lyse der Bakterienzellwand bewirken (z. B. Proteinasen, Alkali) aufgeschlossen. Resultat der diversen Vorbehandlungen ist immer eine Probenflüssigkeit, welche Nukleinsäuren gelöst enthält und in der evtl. die in den vorbereitenden Schritten eingesetzten Reagenzien sowie gegebenenfalls zerstörte Zellbestandteile enthalten sind.

Ist die Konzentration oder Menge der Nukleinsäure in der Probe, gegebenenfalls nach Probenvorbereitung noch zu gering für einen sinnvollen Nachweis, so kann sich ein Amplifikationsverfahren anschließen. Solche Verfahren sind dem Fachmann bekannt, z. B. die PCR oder NASBA (EP-A-0 302 822). Sie dienen dazu, eine ausreichende Menge an nachzuweisenden Nukleinsäuren zu produzieren, aber auch, das Verhältnis von nachzuweisenden Nukleinsäuresequenzen zu nicht nachzuweisenden Nukleinsäuresequenzen zu erhöhen. Sie erhöhen also auch die Spezifität des Nachweises.

Im erfindungsgemäßen Verfahren wird die Probe, welche die nachzuweisende Nukleinsäure oder davon abgeleitete Nukleinsäuren, wie die Resultate einer Amplifikationsreaktion, also Amplifikate, enthält, mit einer Probe in Kontakt gebracht, welche die nachzuweisende Nukleinsäure über einen nukleinsäurebindenden Rest binden kann, aber auch eine weitere chemische Gruppe enthält, die selbst nicht an die nachzuweisende Nukleinsäure binden kann. Die Bindung der Sonde findet bevorzugt über die bekannten Wasserstoffbrückenwechselwirkungen zwischen komplementären Basen der nachzuweisenden Nukleinsäure und dem nukleinsäurebindenden Rest statt (Watson-Crick oder/und Hoogsteen-Basenpaarungen).

Daher handelt es sich bei dem nukleinsäurebindenden Rest bevorzugt um ein Grundgerüst (Backbone), an welches eine Sequenz von natürlich vorkommenden oder künstlichen Basen gebunden ist, die zu einer Basensequenz der nachzuweisenden Nukleinsäure für eine Bindung ausreichend komplementär sind. Die Komplementarität wird sich auch danach bemessen, wie spezifisch der Nachweis sein soll. So wird bei Bedingungen geringer Stringenz möglicherweise eine Reihe von Nukleinsäuren mit unterschiedlicher Sequenz in dem Bereich der Hybridisierung mit Sondenmolekülen hybridisieren, obwohl keine vollständige Komplementarität gegeben ist, d. h. ein oder mehr Basen der Sondensequenz eine andere als die hierzu komplementäre Base auf der Nukleinsäure vorfinden. Auch bezüglich der Länge wird sich der nukleinsäurebindende Rest an der erwünschten Spezifität und der Stringenz orientieren.

Beispiele für nukleinsäurebindende Reste sind Oligonukleotide und künstliche Oligomere. Im Sinne der vorliegenden Erfindung sind Oligomere mit einem natürlich nicht vorkommenden Backbone bevorzugt, wobei in dem Backbone auch natürlich vorkommende Bausteine, z. B. Mononukleotide, enthalten kann. Besonders bevorzugt sind solche Backbones, bei denen eine oder mehrere der Monomereinheiten über Peptidbindungen miteinander verknüpft sind.

Ausgezeichnet eignen sich beispielsweise die sogenannten Peptidnukleinsäuren (PNA), wie sie in WO 92/20702 beschrieben sind. Da ihre Bindung zu komplementären Nukleinsäuresequenzen auch unter Niedrigsalzbedingungen sehr stabil sind, ist mit ihnen eine gute Differenzierung von evtl. möglichen Nukleinsäure-Nukleinsäure-Doppelsträngen möglich.

Ein weiterer Vorteil von PNA, der bei der vorliegenden Erfindung zum Tragen kommen kann, ist die vereinfachte Synthese von Konjugaten aus einem oder mehreren nukleinsäurebindenden Resten und einem oder mehreren nicht nukleinsäurebindenden Peptiden.

Das Konjugat enthält ferner mindestens eine chemische Gruppe, die nicht an der Bindung des Konjugats mit der Nukleinsäure beteiligt ist, die aber bei Bindung des Konjugats an die Nukleinsäure eine neue, nachweisbare Eigenschaft erhält. Diese chemische Gruppe kann bevorzugt aus der Gruppe Peptide, Hormone und Antigene ausgewählt werden. Bevorzugt sind Peptide. Das Molekulargewicht der Gruppe ist vorzugsweise größer als 1.000 D, besonders bevorzugt liegt es bei zwischen 2.000 und 10.000 D. Die Gruppe kann auch solche Bauteile, Atome enthalten, die für die Eigenschaft keine besondere Rolle spielen, wie z. B. Linker, die die eigentliche Gruppe mit dem nukleinsäurebindenden Rest verbinden. Die Verbindung des nukleinsäurebindenden Rests mit der Gruppe ist bevorzugt kovalent, z. B. über eine Amidbindung. Beispiele für eine neue Eigenschaft sind z. B. Strukturänderungen, insbesondere geometrische Anordnung, z. B. Änderung der Konformation oder auch der Linearität, wie bei Peptidketten, die in eher gestreckter oder in gefalteter Form vorliegen können, oder auch die Fähigkeit, von einem Bindepartner erkannt zu werden, oder diesen zu erkennen, die Reaktivität in chemischen oder enzymatischen Reaktionen oder die Fähigkeit zur Übertragung von Energieeinheiten zur Erzeugung definierter Anregungszustände.

Bevorzugt im Sinne der Erfindung sind Gruppen, deren Konformation durch Bindung des Konjugats an die Nukleinsäure so verändert wird, daß sie im an die Nükleinsäure hybridisierten Zustand, im Gegensatz zu derselben Gruppe in einem Konjugatmolekül, das nicht an die Nukleinsäure gebunden ist, nicht mehr von einem Bindepartner erkannt und gebunden werden können. Beispiele für solche Gruppen sind für sich alleine nicht reaktive Teilkomponenten eines enzymatischen Komplexes, wie z. B. sogenannte Enzymdonoren. Typisches und bevorzugtes Beispiel eines aus für sich alleine nicht enzymatisch aktiven Teilkomponenten aufgebauten Enzymkomplexes ist β-Galaktosidase, wie in Clin. Chem. 32, 9, 1637 - 1641 (1986) beschrieben. Für den Fall, daß ein Konjugat aus PNA und dem dort beschriebenen Enzymdonors an eine Nukleinsäure gebunden wird, ändert sich die Konformation des Enzymdonors, so daß die Affinität zum Enzymakzeptor reduziert ist, und sich im günstigsten Fall überhaupt kein aktiver Enzymkomplex bilden kann.

Die Änderung der Konformation kann gegenüber einem Konjugat aus einem nukleinsäurebindenden Rest und einer chemischen Gruppe oft erheblich gesteigert werden, wenn sie mit Hilfe eines weiteren nukleinsäurebindenden Restes stabilisiert wird, der die nachzuweisende Nukleinsäure an einer anderen, von der Bindungsposition des ersten Restes entfernten Stelle an dieselbe Nukleinsäure bindet. Die Entfernung zwischen den beiden Bindungsstellen wird hierzu so gewählt, daß die chemische Gruppe die Distanz noch überbrücken kann, sie sich aber von der normalen Distanz der Verknüpfungspunkte im Konjugat unterscheidet. Somit findet bei Anwesenheit der nachzuweisenden Nukleinsäure in der Probe eine Linearisierung der chemischen Gruppe statt, wodurch sich z. B. deren Bindeeigenschaften ändern.

Die Verknüpfungspunkte der chemischen Gruppe mit dem nukleinsäurebindenden Rest werden so gewählt, daß eine Bindung des Konjugats an die Nukleinsäure dadurch nicht verhindert wird. Als zweckmäßig hat sich eine Verknüpfung der Enden des Rests/der Reste und der Gruppe erwiesen, wodurch auch in der Synthese Vereinfachungen möglich werden.

Bei der Behandlung der Probe mit dem Konjugat wird im Sinne der Erfindung unter den für Hybridisierungstests bzw. aus WO 92/20703 bekannten Bedingungen ein Hybrid aus Konjugat und nachweisender Nukleinsäure gebildet, während ein eventuell im Überschuß gegenüber der Menge an nachzuweisender Nukleinsäure eingesetzter Teil des Konjugats ungebunden verbleibt. Gemäß der vorliegenden Erfindung wird nun entweder der gebundene oder der ungebundene Anteil des Konjugats aufgrund der Eigenschaft bestimmt. Im bevorzugten Fall des Nachweises des ungebundenen Anteils wird das Meßsignal gegenüber einem Vergleichsfall, bei dem die nachzuweisende Nukleinsäure in geringerem Maß oder gar nicht vorhanden ist. moduliert, insbesondere reduziert sein, entsprechend der Relation von freiem zu gebundenem Konjugat. Hieraus kam auf die Menge oder die Anwesenheit der nachzuweisenden Nukleinsäure geschlossen werden. Bei Aufnähme einer Eichkurve. z. B. durch Erfassung der Meßsignale bei mehreren bekannten Konzentrationen, sind sogar quantitative Bestimmungen möglich.

Die Bestimmung richtet sich im wesentlichen nach der Eigenschaft, die durch die Bindung geändert wurde. So ist es im Fall der Verhinderung der Bindung eines aktiven Enzymkomplexes möglich, die Enzymaktivität zu bestimmen. Dies geschieht zweckmäßigerweise über ein Substrat des Enzyms, welches eine einfach zu detektierende Reaktion eingeht, z. B. die enzymatische Spaltung zu einem gefärbten, fluoreszierenden oder lumineszenten Produkt. Die Enzymreaktion kann entsprechend in Photometern oder Fluorometern verfolgt werden.

Das erfindungsgemäße Verfahren hat den Vorteil, daß es keine heterogene Versuchsführung erforderlich macht, d. h. daß eine Abtrennung der gebildeten Hybride von einem evtl. vordenen Überschuß an Sonde (Konjugat) entfallen kann. Die Verfahrensführung kann homogen, d. h. ohne Abtrennschritt erfolgen.

In Fig. 1 ist eine bevorzugte Ausführungsform des erfindungsgemäßen Verfahrens gezeigt, bei dem DNA mit Hilfe eines Chimären aus 2 PNA-Teilen (PNA) und einem Enzymdonorteil (ED) nachgewiesen wird. Im Falle der Anwesenheit der DNA kann der Enzymakzeptor (EA) nicht in der üblichen Weise an den Donor binden, wodurch das aktive Enzym (E) nicht oder in geringerem Maße gebildet wird. Die Umsetzungsrate eines Enzymsubstrats wird reduziert. Nicht gebundene Sonde (sei es ein Überschuß oder bei Abwesenheit von DNA) reagiert mit EA zum Enzym E, welches ein Substrat zum Produkt umsetzt. Die Produktbildung wird gemessen und ist proportional zur Menge an ungebundener Sonde.

Ebenfalls Gegenstand der Erfindung ist ein Reagenz zum Nachweis von Nukleinsäuren, enthaltend ein Konjugat aus einer chemischen Gruppe, welche selbst nicht an die nachzuweisende Nukleinsäure binden kann. aber bei Bindung des Konjugats an die Nukleinsäure eine neue, nachweisbare Eigenschaft erhält, und einem nukleinsäurebindenden Rest. Hierbei gelten die für das Verfahren genannten Bevorzugungen bezüglich des Konjugats.

Ebenfalls Gegenstand der Erfindung ist ein Reagenzkit zum Nachweis von Nukleinsäuren enthaltend vorzugsweise in getrennten Behältern ein erfindungsgemäßes Reagenz sowie Reagenzien zur Bestimmung der neuen nachweisbaren Eigenschaft.

Die folgenden Beispiele erläutern die Erfindung näher.

**Beispiele**

Beispiel 1:

Synthese eines Konjugats (Chimären) (PNA-ED(AS 5-51)-PNA)

H-TTGTCTCGATGAGAG-Gly-Ser-Leu-Ala-Val-Val-Leu-Gln-Arg-Arg-Asp-Trp-Glu-Asn-Pro-Gly-Val-Thr-Gln-Leu-Asn-Arg-Leu-Ala-Ala-His-Pro-Pro-Phe-Ala-Ser-Trp-Arg-Asn-Ser-Glu-Glu-Ala-Arg-Thr-Asp-Arg-Pro-Ser-Gln-Gln-Leu-CAC-TATGGAACTCTG-NH$_2$

Die PNA-Fragmente hybridisieren an ein 235 bp DNA Amplicon, welches aus einem 7.5 kb Chlamydia trachomatis Plasmid erhalten wurde. Die zu den beiden PNA-Teilen komplementären DNA-Abschnitte liegen 100 bp auseinander.

Primer 1: 5'-CTGTAACAACAAGTCAGGTTGCGC-3' (SEQ ID NO 1)
Primer 2: 5'-GTACTAGAGGACTTACCTCTTCCC-3' (SEQ ID NO 2)

Die Synthese erfolgte auf einem ABI 433A Peptid-Synthesizer der Firma Applied Biosystems. Die Synthesen wurden im 5 µmol scale in einem 3 ml Reaktionsgefäß durchgeführt, ein kleineres Measuring Loop (150 µl) wurde verwendet. Die Monomerbausteine (PNA-Bausteine: Boc-T-OH, Boc-A(Z)-OH, Boc-C(Z)-OH und Boc-G(Z)-OH, erhältlich von der Fa. Perseptive Biosystems; Aminosäurederivate: Boc-Leu-OH, Boc-Gln-OH, Boc-Ser(Bzl)-OH x DCHA, Boc-Pro-OH, Boc-Arg(Tos)-OH, Boc-Asp(OBzl)-OH, Boc-Thr(Bzl)-OH, Boc-Ala-OH, Boc-Glu(OBzl)-OH, Boc-Asn-OH, Boc-Trp(formyl)-OH, Boc-His(Tos)-OH, Boc-Val-OH, Boc-Gly-OH, erhältlich von der Fa. Novabiochem AG) wurden in NMP gelöst und in individuelle Kartuschen eingespritzt (140 µl, 0.26M). Das mit Boc-Gly derivatisierte MBHA-Trägermaterial wird in das 3 ml Reaktionsgefäß gefüllt und am Synthesizer angebracht. Trifluoressigsäure/m-Cresol 95:5 (2 x 180 sec); Flaschenposition 2) wird verwendet, um die Boc-Schutzgruppe abzuspalten. Je 2 Waschmodule (Dichlormethan- und NMP-Modul bestehend jeweils 5 aufeinanderfolgenden Waschschritten) befinden sich zwischen Boc-Abspaltung und dem Kupplungsmodul (Dichlormethan = Flaschenposition 9; NMP = Flaschenposition 10). 140 µl 0.26 M Monomer (36 µmol), 150 µl 0.21 M HATU (O-(7-Azabenzotriazol-1-yl)-1,1,3,3-tetramethyluroniumhexafluorophosphat) in NMP (32 µmol) und 150 µl 0.5 M Diisopropylethylamin in NMP (75 µmol) werden in jedem Kupplungsschritt gefördert (Diisopropylethylamin-Lösung = Flaschenposition 7; HATU-Lösung = Flaschenposition 8). Die Monomere werden in der Synthesekartusche voraktiviert (1 min), dann in das Reaktionsgefäß überführt. Die Kupplungszeit beträgt 10 min, die Monomerenkonzentration während der Kupplung beträgt 0.08 M. Nach dem Kupplungsmodul erfolgt ein Capping-Modul mit Essigsäureanhybrid/NMP/Pyridin 1:25:25 (1 min; Flaschenposition 4). Jeder Synthesecyclus wird mit einem NMP Waschmodul abgeschlossen. Nach dem letzten Synthesecyclus folgt ein Dichlormethan-Waschmodul, um das Harz zu trocknen. Die Kupplungen wurden regelmäßig durch einen qualitativen Kaiser-Test kontrolliert, nach unzureichendem Kaiser-Test erfolgte Doppelkupplung.

Die Abspaltung des PNA-Peptid-Chimärs vom Harz und der Schutzgruppen erfolgt in einem manuellen Schritt außerhalb des Gerätes in einer speziellen, verschließbaren Glasfritte. Das Harz wird zuerst mit Trifluoressigsäure gewaschen, dann schüttelt man 1.5 Stunden mit 2 ml Trifluormethansulfonsäure/Trifluoressigsäure/m-Cresol 2:8:1. Die Abspaltlösung wird in ein Zentrifugenglas gesaugt. Man wäscht mit 1 ml Trifluoressigsäure nach und präzipitiert die PNA mit Diethylether. Das Präzipitat wird nach Zentrifugation von der überstehenden Lösung getrennt. Danach wäscht man zweimal mit Diethylether.

Das PNA-Peptid-Chimär wurde über eine analytische RP18-HPLC (DeltaPak, Waters, 5 µ, 125 x 4 mm) mit einem Wasser/Acetonitril/0. 1% Trifluoressigsäure-Gradienten bei 60°C analysiert.

Das Chimär wurde über eine präparative RP18-HPLC (Nucleosil-RP18/Macherey-Nagel, 5µ, 250 x 20 mm) mit einem Wasser/Acetonitril/0,1% Trifluoressigsäure-Gradienten bei 60°C bis zur chromatographischen Einheitlichkeit gereinigt.

Die Analyse des gereinigten PNA-Peptid-Chimärs wird mit MALDI-TOF-MS durchgeführt.

Beispiel 2:

Nachweis eines 235 bn DNA Amplicons aus der Genom-Sequenz von Chlamydia trachomatis, erhalten aus einem 7.5 kb Plasmid

Folgende Lösungen werden verwendet:

A: Phospat-Puffer          0.1 m Kaliumphosphat/0.1 Natriumphosphat pH 7.0
B: PEP 10 nM               in A
C: Amplicon 1 nM           in A
D: Amplicon 10 nM          in A
E: Amplicon 100 nM         in A
F: Enzym Akzeptor 1.2 mM   in A
G: Chlorphenolrot-Galactosid   (CPRG) 4 mg/ml in A + 0.1 % Tween 20

500 µl Lösung B wird mit a) 500 µl A, b) 500µl Lösung C, c) 500µl Lösung D d) 500 µl Lösung E vermischt und 30 min bei 37°C inkubiert. Dann wird jeweils 10 µl von Reagenz E und F zugegeben, vermischt und die Optische Dichte OD 1 min, 2 min und 5 min nach Zugabe gemessen. Die Werte werden in ein Diagramm eingetragen ( x = t, y = OD ).

Für a)-c) ergeben sich Geraden $y = c_1 + k \cdot x$          mit

k =    a) 1.55
       b) 1.20
       c) 0.65
       d) 0.35

Diese Werte ergeben eine Eichkurve zur Ermittlung einer unbekannten Konzentration $c_{Amplicon}$ in einer Probenlösung.

**Patentansprüche**

1. Verfahren zum Nachweis einer Nukleinsäure in einer Probe enthaltend die Schritte

   - Behandlung der Probe mit einem Konjugat aus einer chemischen Gruppe, welche selbst nicht an die nachzuweisende Nukleinsäure bindet, aber bei Bindung des Konjugats an die Nukleinsäure eine neue, nachweisbare Eigenschaft erhält, und einem nukleinsäurebindenden Rest unter Bedingungen, bei denen das Konjugat an die nachzuweisende Nukleinsäure bindet, und

   - Bestimmung der neuen Eigenschaft der chemischen Gruppe als Zeichen der Anwesenheit oder der Menge der nachzuweisenden Nukleinsäure in der Probe.

2. Verfahren gemäß Anspruch 1, dadurch gekennzeichnet, daß zur Bestimmung der neuen Eigenschaft die behandelte Probe mit einem Bindepartner der chemischen Gruppe inkubiert wird, welcher zwar an das nicht an die Nukleinsäure gebundene Konjugat, nicht jedoch oder in reduziertem Maße an das an die Nukleinsäure gebundene Konjugat binden kann.

3. Verfahren gemäß einem der vorangehenden Ansprüche, dadurch gekennzeichnet, daß die neue Eigenschaft eine neue geometrische Anordnung der Bestandteile der chemischen Gruppe ist.

4. Verfahren gemäß einem der vorangehenden Ansprüche, dadurch gekennzeichnet, daß der nukleinsäurebindende Rest eine Peptidnukleinsäure enthält.

5. Verfahren gemäß einem der vorangehenden Ansprüche, dadurch gekennzeichnet, daß die chemische Gruppe eine für sich alleine nicht reaktive Teilkomponente eines enzymatischen Komplexes ist.

6. Verfahren gemäß einem der vorangehenden Ansprüche, dadurch gekennzeichnet, daß der nukleinsäurebindende Rest ein Antigen enthält.

7. Verfahren gemäß einem der vorangehenden Ansprüche, dadurch gekennzeichnet, daß der nukleinsäurebindende Rest ein Peptid enthält.

8. Verfahren gemäß einem der vorangegangenen Ansprüche, dadurch gekennzeichnet, daß es sich bei dem Peptid um eine Teilsequenz der $\beta$-Galaktosidase handelt.

9. Verfahren gemäß einem der vorangehenden Ansprüche, dadurch gekennzeichnet, daß das Konjugat zwei voneinander durch die chemische Gruppe getrennte nukleinsäurebindende Reste enthält.

10. Verfahren gemaß einem der vorangehenden Ansprüche, dadurch gekennzeichnet, daß es sich um ein homogenes Nachweisverfahren handelt.

11. Reagenz zum Nachweis von Nukleinsäuren, enthaltend ein Konjugat aus einer chemischen Gruppe, welche selbst nicht an die nachzuweisende Nukleinsäure binden kann, aber bei Bindung des Konjugats an die Nukleinsäure eine neue, nachweisbare Eigenschaft enthält, und einem nukleinsäurebindenden Rest.

12. Reagenzkit zum Nachweis von Nukleinsäuren, enthaltend ein Reagenz gemäß Anspruch 11 sowie Reagenzien zur Bestimmung der neuen nachweisbaren Eigenschaft.

Fig. 1